# EUROPEAN PATENT APPLICATION

(11) **EP 3 006 011 A1**
(43) Date of publication of application: **13.04.2016**
(21) Application number: 13886001.0
(22) Date of filing: 28.05.2013
(51) Int. Cl.: A61K 6/033, A61C 5/08, A61C 13/083, A61L 27/00

(54) **COMPOSITION TO BE FIXED IN ORAL CAVITY**

(71) Applicant: Fujitsu Limited, Kawasaki-shi, Kanagawa 211-8588 (JP)
(72) Inventor: WAKAMURA, Masato, Kawasaki-shi Kanagawa 211-8588 (JP); ANAZAWA, Toshihisa, Kawasaki-shi Kanagawa 211-8588 (JP); TSUKADA, Mineharu, Kawasaki-shi Kanagawa 211-8588 (JP); COORAY, Nawalage Florence, Kawasaki-shi Kanagawa 211-8588 (JP)
(74) Representative: Schultes, Stephan
(86) International application number: PCT/JP2013/064731
(87) International publication number: WO 2014/192075

(57) **Abstract**

An intraoral fixing composition, containing: titanium apatite, which is obtained by substituting part of calcium in calcium hydroxyapatite with titanium, wherein the intraoral fixing composition is fixed at an intraoral site in an unreleasable state.

## Description

### Technical Field

The embodiment discussed herein relates to an intraoral fixing composition, which is used by fixing at an intraoral site.

### Background Art

Calcium hydroxyapatite (calcium phosphate) that constitutes teeth of the mammals is weak to acids, and is easily dissolved. Therefore, tooth decay occurs due to acids secreted by Streptococcus mutans living inside a mouth of a human. As for a treatment method of tooth decay, there is a method where a decayed part of a tooth is drilled out, and reinforced with a filler, or a crown (overlay).

However, a number of Streptococcus mutans in a mouth cannot be reduced, and tooth decay cannot be prevented merely by reinforcing a decayed part of a tooth. Therefore, oral cleaning is generally performed. The oral cleaning is typically performed by brushing using a tooth brush. However, a cleaning effect by tooth brushing is not sufficient, if a tooth brush is stained with funguses.

Therefore, the present inventors have proposed a tooth brushing tool, in which a material having a photocatalytic activity and an ability of adsorbing organic matter is kneaded in a brush part, or a surface of the brush part is coated with the material (see PTL 1). According to the proposed technology, funguses attached to the tooth brushing tool can be simply and effectively decomposed and removed.

However, there is a large difference in a state for performing a cleaning method with a tooth brushing tool between individuals, and there are not so many people who can carry out a proper cleaning method. Therefore, desired is an intraoral fixing composition, which is fixed at an intraoral site, and can directly sterilize Streptococcus mutans inside a mouth.

As for the intraoral fixing composition fixed at an intraoral site, moreover, desired is a material, which is not easily dissolved with acids secreted by Streptococcus mutans, and has excellent acid resistance.

Accordingly, it is current situation that there is a need for an intraoral fixing composition, which is fixed at an intraoral site, can directly sterilize Streptococcus mutans inside a mouth, and has excellent acid resistance.

### Citation List

### Patent Literature

PTL 1 Japanese Patent Application Laid-Open (JP-A) No. 2010-125067

### Summary of Invention

### Technical Problem

The disclosed embodiments aims to solve the aforementioned various problems in the art, and to achieve the following object. Specifically, an object of the disclosed embodiments is to provide an intraoral fixing composition, which is fixed at an intraoral site, can directly sterilize Streptococcus mutans inside a mouth, and has excellent acid resistance.

### Solution to Problem

The means for solving the aforementioned problems are as follows.

The disclosed intraoral fixing composition contains: titanium apatite, which is obtained by substituting part of calcium in calcium hydroxyapatite with titanium, wherein the intraoral fixing composition is fixed at an intraoral site in an unreleasable state.

### Advantageous Effects of Invention

The disclosed intraoral fixing composition can solve the aforementioned various problems in the art, and can provide an intraoral fixing composition, which is fixed at an intraoral site, can directly sterilize Streptococcus mutans inside a mouth, and has excellent acid resistance.

### Brief Description of Drawings

FIG. 1 is a perspective view depicting one example of a crown.
FIG. 2 is a cross-sectional view depicting one example of a crown.
FIG. 3 is a perspective view depicting one example of a tooth having a defective part.
FIG. 4 is a perspective view depicting a state where an inlay is fitted to the tooth having a defective part, which is depicted in FIG. 3.
FIG. 5 is a graph depicting a survival rate of streptococcus mutans.

### Description of Embodiments

### (Intraoral Fixing Composition)

The disclosed intraoral fixing composition contains titanium apatite, which is obtained by substituting part of calcium in calcium hydroxyapatite with titanium.

The intraoral fixing composition is fixed at an intraoral site in an unreleasable state. In the present specification, "unreleasable" means that the intraoral fixing composition cannot be released by a will of a person having the intraoral fixing composition fixed at her or his intraoral site. In other words, the intraoral fixing composition is an intraoral fixing composition, which is fixed at an intraoral site as a treatment applied by a dentist.

Note that, the intraoral fixing composition is also called a dental prosthesis.

It has been known that titanium apatite, which is obtained by substituting part of calcium in calcium hydroxyapatite with titanium, has a function as a photocatalyst. However, the known photocatalytic function of the titanium apatite is under a typical environment. It has not been know that the titanium apatite exhibits a photocatalytic function in an intraoral environment.

As a result of the researches diligently performed by the present inventors, the present inventors have found that the titanium apatite exhibits a photocatalytic function in a low pH environment created by acids secreted by Streptococcus mutans living inside a mouth of a human, to decompose the Streptococcus mutans.

Moreover, the present inventors have found that the titanium apatite excels in acid resistance compared to calcium hydroxyapatite.

Calcium hydroxyapatite is easily dissolved with any acid. As a result of the researches performed by the present inventors, the titanium apatite is not dissolved with typical acids, and is dissolved only with hot concentrated sulfuric acid of 200°C.

The intraoral fixing composition is appropriately selected depending on the intended purpose, provided that it is an intraoral fixing composition fixed at an intraoral site in an unreleasable state. Examples of the intraoral fixing composition include inlays, crowns, and implant dentures.

The inlay and the crown are restorations adjusted to a shape suitable for a cavity or an abutment tooth, after forming the cavity or the abutment tooth. In the case where a defective part of a tooth is small, the tooth is typically restored with an inlay. In the case where collapse of a tooth is large, and an original shape of the tooth cannot be reproduced with an inlay, the tooth is restored by covering the entire tooth with a crown. The crowns include bridges.

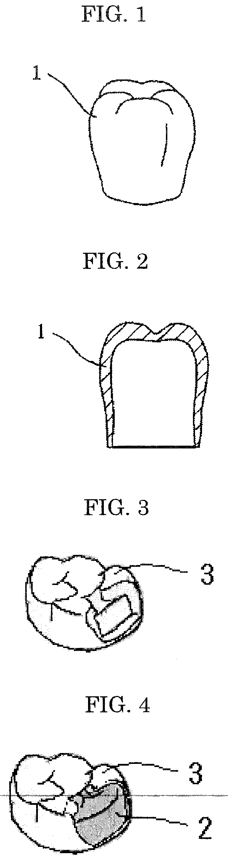

One example of the crown is depicted in FIGs. 1 and 2. In FIGs. 1 and 2, a referential sign 1 denotes a crown.

A perspective view of a tooth having a defective part is depicted in FIG. 3. A drawing where an inlay is fitted to the defective part of the tooth of FIG. 3 is depicted in FIG. 4. In FIGs. 3 and 4, a referential sign 3 denotes a tooth. In FIG. 4, a referential sign 2 denotes an inlay.

The implant denture is a denture, which is prepared by inserting an implant fixture into a site of a jawbone above which a tooth is missing, and fixing the denture to the implant fixture.

Moreover, examples of the intraoral fixing composition include a composite resin formed by filing a cavity of a tooth.

### <Titanium Apatite>

The intraoral fixing composition contains titanium apatite, which is obtained by substituting part of calcium in calcium hydroxyapatite with titanium.

The calcium hydroxyapatite (CaHAP) contains calcium atoms (Ca) having excellent adsorptivity, and phosphorus atoms (P) having excellent biocompatibility. For example, the calcium hydroxyapatite is represented by Ca₁₀(PO₄)₆(OH)₂.

A photocatalytic partial structure capable of exhibiting a photocatalytic function is formed in a crystal structure of the titanium apatite by incorporating (by substitution) Ti (titanium) in the crystal structure as part of metal atoms constituting the crystal structure of the calcium hydroxyapatite (CaHAP), for example by substituting part of Ca (calcium) sites with Ti (titanium).

The average particle diameter of the titanium apatite is appropriately selected depending on the intended purpose, and the average particle diameter of the titanium apatite is preferably 5 µm or smaller.

When the average particle diameter of the titanium apatite is greater than 5 µm, the kneading state into the intraoral fixing composition or the surface coating state thereof may not be suitably maintained.

The titanium apatite for use may be synthesized, or selected from commercial products. Examples of the commercial products thereof include calcium·titanium hydroxyapatite (TiHAP; PHOTOHAP PCAP-100, manufactured by TAIHEI CHEMICAL INDUSTRIAL CO., LTD.).

An amount of the titanium apatite in the intraoral fixing composition is appropriately selected depending on the intended purpose.

It is preferred that the titanium apatite be kneaded into the intraoral fixing composition, or a surface of the intraoral fixing composition be coated with the titanium apatite.

The kneading method is appropriately selected depending on the intended purpose, and examples of the kneading method include a method, in which the titanium apatite is kneaded with materials of an intraoral fixing composition to form an intraoral fixing composition.

The surface coating method is appropriately selected depending on the intended purpose, and examples of the surface coating method include a method, in which a shaped intraoral fixing composition is covered with the titanium apatite through a physical method. As for the physical method, ion deposition, sputtering, or coating can be applicable.

The intraoral fixing composition is fixed at an intraoral site. In the state where the intraoral fixing composition is fixed at the intraoral site, ultraviolet rays are applied to the intraoral fixing composition, so that the titanium apatite exhibits a photocatalytic function in an intraoral environment to decompose Streptococcus mutans.

A method for applying ultraviolet rays to the intraoral fixing composition inside a mouth is appropriately selected depending on the intended purpose. Examples of the method include a method, in which ultraviolet rays are applied to the intraoral fixing composition inside a mouth using a light-emitting diode that emits ultraviolet rays. In the case where it is desired not to apply ultraviolet rays to sites inside a mouth other than the intraoral fixing composition, the sites other than the intraoral fixing composition in the mouth may be covered with an aluminum foil.

Since the intraoral fixing composition has a photocatalytic function, the intraoral fixing composition can be suitably used as a dental prosthesis for preventing tooth decay. Since the intraoral fixing composition excels in acid resistance, moreover, Streptococcus mutans inside a mouth can be sterilized over a long period.

### Examples

The intraoral fixing composition is more specifically explained through Examples hereinafter, but the disclosed intraoral fixing composition is not limited to these examples.

### (Experimental Example 1)

As for titanium apatite, calcium·titanium hydroxyapatite (TiHAP; PHOTOHAP PCAP-100, manufactured by TAIHEI CHEMICAL INDUSTRIAL CO., LTD., a white powder having particle diameters of 3 µm to 8 µm) was used.

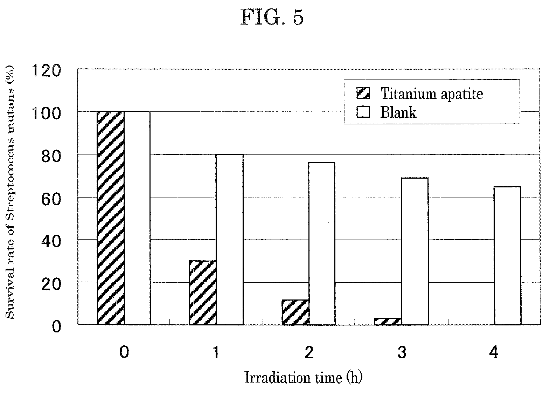

A clear container housing therein a culture fluid containing 3.0 × 10⁵ Streptococcus mutans (adjusted to pH 4.0 with lactic acid: assuming an intraoral environment) was charged with 1.0 g of the titanium apatite. Ultraviolet rays were applied thereto by means of a black light (0.5 mW/cm²), to determine a survival rate of the Streptococcus mutans. The result is presented in FIG. 5.

Moreover, ultraviolet rays were also applied to a blank sample, in which titanium apatite was not added to a culture fluid containing 3.0 × 10⁵ Streptococcus mutans (adjusted to pH 4.0 with lactic acid: assuming an intraoral environment) by means of a black light (0.5 mW/cm²), to determine a survival rate of the Streptococcus mutans. The result is presented in FIG. 5.

The survival rate of the Streptococcus mutans in the blank sample, in which no titanium apatite was added to the culture fluid, was 65% after 4 hours of the ultraviolet ray irradiation, compared to the initial amount. In the case where the titanium apatite was added to the culture fluid, the survival rate of the Streptococcus mutans was reduced 30% from the initial amount with 1 hour of the ultraviolet ray irradiation, and the survival rate of the Streptococcus mutans became 0% with 4 hours of the ultraviolet ray irradiation.

It was confirmed from the results above that the titanium apatite could decompose Streptococcus mutans in the intraoral low pH environment.

### (Experimental Example 2)

A dissolved amount of calcium·titanium hydroxyapatite was measured by dipping 1.0 g of calcium·titanium hydroxyapatite (TiHAP; PHOTOHAP PCAP-100, manufactured by TAIHEI CHEMICAL INDUSTRIAL CO., LTD., a white powder having particle diameters of 3 µm to 8 µm) in 10 mL of an acidic aqueous solution (corresponding to an intraoral environment with acids released from STReptococcus mutans) of 36°C, which was adjusted to pH 4.0 with lactic acid. One hour later, the dissolved amount was 6% by mass.

A dissolved amount of calcium hydroxyapatite was measured in the same manner to the above, provided that the calcium·titanium hydroxyapatite was replaced with calcium hydroxyapatite (HAP100, manufactured by TAIHEI CHEMICAL INDUSTRIAL CO., LTD.). One hour later, the dissolved amount was 45% by mass.

It was confirmed from the results above that the titanium apatite exceled in acid resistance in the intraoral environment.

### (Example 1)

A hybrid ceramic crown (a hybrid of a ceramic and a resin), in which calcium·titanium hydroxyapatite (TiHAP; PHOTOHAP PCAP-100, manufactured by TAIHEI CHEMICAL INDUSTRIAL CO., LTD., a white powder having particle diameters of 3 µm to 8 µm) had been kneaded, was provided.

A survival rate of Streptococcus mutans in an intraoral low pH environment was measured in the same manner as in Experimental Example 1, provided that 1.0 g of the titanium apatite was replaced with 1.0 g of the hybrid ceramic crown above.

### (Example 2)

A hybrid ceramic crown (a hybrid of a ceramic and a resin), a surface of which had been coated with calcium·titanium hydroxyapatite (TiHAP; PHOTOHAP PCAP-100, manufactured by TAIHEI CHEMICAL INDUSTRIAL CO., LTD., a white powder having particle diameters of 3 µm to 8 µm), was provided.

Note that, the surface coating was performed by dipping the hybrid ceramic crown in a dispersion liquid, in which 30 parts by mass of the calcium·titanium hydroxyapatite was dispersed in 100 parts by mass of pure water, for 3 minutes, followed by drying for 2 hours in an oven of 100°C.

A survival rate of Streptococcus mutans in an intraoral low pH environment was measured in the same manner as in Experimental Example 1, provided that 1.0 g of the titanium apatite was replaced with 1.0 g of the hybrid ceramic crown above.

### (Comparative Example 1)

A hybrid ceramic crown (a hybrid of a ceramic and a resin) was provided.

A survival rate of Streptococcus mutans in an intraoral low pH environment was measured in the same manner as in Experimental Example 1, provided that 1.0 g of the titanium apatite was replaced with 1.0 g of the hybrid ceramic crown (a hybrid of a ceramic and a resin) above.

### (Comparative Example 2)

A hybrid ceramic crown (a hybrid of a ceramic and a resin), in which calcium hydroxyapatite (HAP100, manufactured by TAIHEI CHEMICAL INDUSTRIAL CO. LTD.) had been kneaded, was provided.

A survival rate of Streptococcus mutans in an intraoral low pH environment was measured in the same manner as in Experimental Example 1, provided that 1.0 g of the titanium apatite was replaced with 1.0 g of the hybrid ceramic crown above.

Comparing the survival rate of Streptococcus mutans upon the application of ultraviolet rays between Example 1, Example 2, Comparative Example 1, and Comparative Example 2, the survival rate of Streptococcus mutans was low in Examples 1 and 2, in which the titanium apatite was used, compared to Comparative Example 1 where the titanium apatite was not used, similarly to the results of Experimental Example 1. Moreover, the survival rate of Streptococcus mutans was low in Examples 1 and 2, in which the titanium apatite was used, compared to Comparative Example 2 where the calcium hydroxyapatite was used.

### Reference Signs List

- 1: crown
- 2: inlay
- 3: tooth

## Claims

1. An intraoral fixing composition, comprising:
titanium apatite, which is obtained by substituting part of calcium in calcium hydroxyapatite with titanium,
wherein the intraoral fixing composition is fixed at an intraoral site in an unreleasable state.

2. The intraoral fixing composition according to claim 1, wherein the titanium apatite is kneaded in the intraoral fixing composition, or a surface of the intraoral fixing composition is coated with the titanium apatite.

3. The intraoral fixing composition according to claim 1 or 2, wherein the intraoral fixing composition is an inlay, a crown, or an implant denture.
